# EUROPEAN PATENT APPLICATION

(11) **EP 4 603 041 A1**
(43) Date of publication of application: **20.08.2025**
(21) Application number: 24191610.5
(22) Date of filing: 30.07.2024
(51) Int. Cl.: A61B 17/88, B25B 15/00

(54) **SCREW REMOVAL DEVICE**

(30) Priority: 16.02.2024 KR 20240022528
(71) Applicant: The Industry & Academic Cooperation in Chungnam National University (IAC), Daejeon 34134 (KR); InnoOrtho Co., Ltd., Sejong-si 30099 (KR); CHUNGNAM NATIONAL UNIVERSITY HOSPITAL, Daejeon 35015 (KR)
(72) Inventor: LEE, Jeong-Kil, 35248 Daejeon (KR); LEE, Seung Hoo, 05658 Seoul (KR)
(74) Representative: BCKIP Part mbB

(57) **Abstract**

A screw removal device includes: a body member including a first groove insertion portion and extending in a front-rear direction; a wedge member including a second groove insertion portion and disposed to be movable in the front-rear direction with respect to the body member; and a pressing member disposed on the body member to move the wedge member in the front-rear direction, wherein a first pressing guide surface is formed in the first groove insertion portion, and a second pressing guide surface is formed in the second groove insertion portion to face the first pressing guide surface, and the wedge member is placed in a disengaged position in which at least one of the first groove insertion portion and the second groove insertion portion are not pushed against each other in a direction perpendicular to at least one of the first pressing guide surface and the second pressing guide surface, or in an engaged position in which the first pressing guide surface and the second pressing guide surface are arranged to face each other, and at least one of the first groove insertion portion and the second groove insertion portion are pushed against each other in the direction perpendicular to at least one of the first pressing guide surface and the second pressing guide surface.

## Description

### TECHNICAL FIELD

The present disclosure relates to a screw removal device.

### BACKGROUND

Recently, instruments commonly used when performing fracture and bone correction surgery in the orthopedic field include a locking compression metal plate and a polygonal locking screw that fixes the locking compression metal plate to the bone. As the polygonal locking screw, screws having a screw head with a hexagonal or star-shaped insertion groove formed thereon are commonly used.

After sufficient bone union has occurred through fracture and bone correction surgery, it may be necessary to remove the polygonal locking screws and the metal plate. Even if bone union does not occur, when circumstances such as infection or metal plate breakage occur, the metal material needs to be removed.

The biggest problem in using the locking compression metal plate and the polygonal locking screw is that a head of the polygonal locking screw is damaged when performing the removal of the metal material.

When the metallic polygonal locking screw is fixed to the bone and bone union proceeds after a certain period of time, the bonding force between the bone and the screw increases. In such cases, when the polygonal locking screw is removed, the high bonding force between the screw and the bone may not be overcome, and thus the polygonal groove formed in the screw head may be crushed.

If the polygonal insertion groove formed in the head of the polygonal locking screw is damaged, it is difficult to remove the screw with a general screwdriver, so that the screw needs to be removed by using a conventional polygonal locking screw removal driver or if this method fails, the screw should be removed after grinding the head of the screw with a special tool (burr).

In the conventional polygonal locking screw removal driver, the polygonal locking screw is removed by coupling the driver to the polygonal insertion groove formed in the head of the polygonal locking screw. In this case, the screw removal driver should be aligned exactly perpendicular to the polygonal insertion groove of the head of the screw and rotated with a certain force and direction to loosen the polygonal locking screw.

In the conventional polygonal locking screw removal driver, when removing the polygonal locking screw, a gap inevitably occurs between the screw removal driver and the polygonal insertion groove of the head of the screw. Due to this gap, the contact force between the screw and the screw removal driver is lowered, which makes it difficult to remove the screw, and the screw removal driver may be damaged after several uses.

In addition, in the conventional polygonal locking screw removal driver, the polygonal insertion groove formed in the head of the screw may be damaged as the contact force is lowered due to the gap between the screw and the screw removal driver. In such cases, in the process of removing the screw head of the screw and the metal plate with a special tool (burr), small metal pieces may remain as foreign substances in a patient's body, which may cause inflammation or infection.

### (Prior Art Document)

### (Patent Document)

Korean Patent Application Publication No. 2021-0006781 (published on January 19, 2021)

### SUMMARY

In view of the above, the present disclosure provides a screw removal device capable of removing a screw from a screw coupling member to which the screw is coupled without damaging an insertion groove formed in a head of the screw, and easily removing the screw from the screw coupling member even if the insertion groove is already damaged.

According to embodiments of the present disclosure, the screw removal device can remove the screw from the screw coupling member to which the screw is coupled without damaging the insertion groove formed in the screw head, and easily remove the screw from the screw coupling member even if the insertion groove is already damaged.

In accordance with a first embodiment of the present disclosure, there is provided a screw removal device including: a body member including a first groove insertion portion and extending in a front-rear direction; a wedge member including a second groove insertion portion and disposed to be movable in the front-rear direction with respect to the body member; and a pressing member disposed on the body member to move the wedge member in the front-rear direction, wherein a first pressing guide surface is formed in the first groove insertion portion, and a second pressing guide surface is formed in the second groove insertion portion to face the first pressing guide surface, and the wedge member is placed in a disengaged position in which at least one of the first groove insertion portion and the second groove insertion portion are not pushed against each other in a direction perpendicular to at least one of the first pressing guide surface and the second pressing guide surface, or in an engaged position in which the first pressing guide surface and the second pressing guide surface are arranged to face each other, and at least one of the first groove insertion portion and the second groove insertion portion are pushed against each other in the direction perpendicular to at least one of the first pressing guide surface and the second pressing guide surface.

Further, in screw removal device, when the first groove insertion portion and the second groove insertion portion are viewed in the front-rear direction with the wedge member placed in the engaged position, the first groove insertion portion and the second groove insertion portion form a polygonal shape.

Further, in screw removal device, the polygonal shape is hexagonal or star-shaped.

Further, in screw removal device, the first pressing guide surface and the second pressing guide surface are inclined such that a first angle, which is an acute angle between a normal to the first pressing guide surface and an imaginary line in the front-rear direction, is greater than a second angle which is an acute angle between a normal to the second pressing guide surface and the imaginary line in the front-rear direction.

Further, in screw removal device, screw removal device, in the body member, a wedge member moving hole which is connected to the first pressing guide surface and in which the wedge member is movably disposed is formed.

Further, in screw removal device, in the body member, an exposed space which is connected to the wedge member moving hole and exposed to an outside and in which the wedge member and the pressing member are movably disposed is formed.

Further, in screw removal device, in the body member, a pressing member moving hole which is connected to the exposed space and in which at least a portion of the pressing member is movably disposed is formed.

Further, in screw removal device, the body member further includes: a member middle portion which is connected to the first groove insertion portion and in which the wedge member moving hole, the exposed space, and a portion of the pressing member moving hole are formed; and a grip portion which is connected to the member middle portion and in which another portion of the pressing member moving hole is formed.

Further, in screw removal device, the wedge member further includes a wedge member movement portion connected to the second groove insertion portion and movably disposed in the wedge member moving hole and the exposed space.

Further, in screw removal device, a moving guide groove, which is connected to the wedge member moving hole and the exposed space and guides the movement of the wedge member movement portion, is further formed in the body member, and a moving guide protrusion movably inserted into the moving guide groove is formed on the wedge member movement portion.

Further, in screw removal device, the moving guide protrusion protrudes radially further than a peripheral surface of the wedge member movement portion.

Further, in screw removal device, the pressing member includes: a pressing movement portion disposed to be movable in the front-rear direction on the body member to press the wedge member; and a handle portion extending from the pressing movement portion to form a predetermined angle with the front-rear direction.

In accordance with a second embodiment of the present disclosure, in screw removal device, a size of the second groove insertion portion is smaller than a size of the first groove insertion portion.

Further, in screw removal device, a first guide surface is further formed in the first groove insertion portion and connected to the first pressing guide surface to form a predetermined angle with the first pressing guide surface, and a second guide surface is further formed in the second groove insertion portion and connected to the second pressing guide surface to form a predetermined angle with the second pressing guide surface, the second guide surface facing the first guide surface.

In accordance with a third embodiment of the present disclosure, there is provided a screw removal device including: a body member including a first groove insertion portion and extending in a front-rear direction; and a wedge member including a second groove insertion portion and disposed to be movable in the front-rear direction with respect to the body member, wherein a first pressing guide surface is formed in the first groove insertion portion, and a second pressing guide surface is formed in the second groove insertion portion to face the first pressing guide surface, and the wedge member is placed in a disengaged position in which at least one of the first groove insertion portion and the second groove insertion portion are not pushed against each other in a direction perpendicular to at least one of the first pressing guide surface and the second pressing guide surface, or in an engaged position in which the first pressing guide surface and the second pressing guide surface are arranged to face each other, and at least one of the first groove insertion portion and the second groove insertion portion are pushed against each other in the direction perpendicular to at least one of the first pressing guide surface and the second pressing guide surface.

Further, in screw removal device, the wedge member is arranged to be selectively connected to at least of the body member, and the body member includes a pressing connection part 121 that presses the wedge member 200 to move in the front-rear direction by moving in the front-rear direction.

### BRIEF DESCRIPTION OF THE DRAWINGS

FIG. 1 shows an exploded perspective view of a screw removal device according to a first embodiment of the present disclosure and an enlarged view of a first groove insertion portion and a wedge member.
FIG. 2 shows a perspective view of the screw removal device according to the first embodiment of the present disclosure when the wedge member is placed in a disengaged position, and an enlarged view and a plan view of the first groove insertion portion and a second groove insertion portion.
FIG. 3 shows a perspective view of the screw removal device according to the first embodiment of the present disclosure when the wedge member is placed in an engaged position, and an enlarged view and a plan view of the first groove insertion portion and the second groove insertion portion.
FIG. 4 is a plan view of the screw removal device in use according to the first embodiment of the present disclosure, showing that the screw removal device has been moved to a location where a screw is coupled to the screw coupling member.
FIG. 5 is a plan view and an enlarged view of the screw removal device in use according to the first embodiment of the present disclosure, showing that the first groove insertion portion of a body member is inserted into an insertion groove of the screw in a state in which the wedge member of the screw removal device is placed in the disengaged position.
FIG. 6 is a plan view and an enlarged view of the screw removal device in use according to the first embodiment of the present disclosure, showing that the wedge member is moved to the engaged position by a pressing member and at least a portion of the first groove insertion portion and at least a portion of the second groove insertion portion are in close contact with at least a portion of the insertion groove of the screw in a state in which the first groove insertion portion of the body member is inserted into the insertion groove of the screw.
FIG. 7 is a plan view of the screw removal device in use according to the first embodiment of the present disclosure, showing that the screw removal device is rotated in a direction that loosens the screw to remove the screw from the screw coupling member in a state in which at least a portion of the first groove insertion portion and at least a portion of the second groove insertion portion are in close contact with at least a portion of the insertion groove of the screw.
FIG. 8 shows a perspective view of a screw removal device according to a second embodiment of the present disclosure and an enlarged view of the first groove insertion portion and the second groove insertion portion.
FIG. 9 shows a perspective view of the screw removal device according to the second embodiment of the present disclosure when the wedge member is placed in the disengaged position, and an enlarged view and a side view of the first groove insertion portion and a second groove insertion portion.
FIG. 10 shows a perspective view of the screw removal device according to the second embodiment of the present disclosure when the wedge member is placed in the engaged position, and an enlarged view and a side view of the first groove insertion portion and the second groove insertion portion.
FIG. 11 is a side view and an enlarged view of the screw removal device in use according to the second embodiment of the present disclosure, showing that the first groove insertion portion of the body member is inserted into the insertion groove of the screw in a state in which the wedge member of the screw removal device is placed in the disengaged position.
FIG. 12 is a side view and an enlarged view of the screw removal device in use according to the second embodiment of the present disclosure, showing that the wedge member is moved to the engaged position by the pressing member and at least a portion of the first groove insertion portion and at least a portion of the second groove insertion portion are in close contact with at least a portion of the insertion groove of the screw in a state in which the first groove insertion portion of the body member is inserted into the insertion groove of the screw.
FIG. 13 shows a perspective view of a screw removal device according to a third embodiment of the present disclosure.
FIG. 14 shows a perspective view of the screw removal device according to the third embodiment of the present disclosure when the wedge member is placed in the disengaged position, and an enlarged view and a side view of the first groove insertion portion and a second groove insertion portion.
FIG. 15 shows a perspective view of the screw removal device according to the third embodiment of the present disclosure when the wedge member is placed in the engaged position, and an enlarged view and a side view of the first groove insertion portion and the second groove insertion portion.
FIG. 16 is a side view and an enlarged view of the screw removal device in use according to the third embodiment of the present disclosure, showing that the first groove insertion portion of the body member is inserted into the insertion groove of the screw in a state in which the wedge member of the screw removal device is placed in the disengaged position.
FIG. 17 is a side view and an enlarged view of the screw removal device in use according to the third embodiment of the present disclosure, showing that at least a portion of the first groove insertion portion and at least a portion of the second groove insertion portion are in close contact with at least a portion of the insertion groove of the screw in a state in which the wedge member of the screw removal device is placed in the disengaged position.

### DETAILED DESCRIPTION

Hereinafter, specific embodiments for implementing the technical ideas of the present disclosure will be described in detail with reference to the drawings.

In addition, in describing the present disclosure, when it is determined that a detailed description of the relevant known configuration or function may obscure the gist of the present disclosure, the detailed description thereof will be omitted.

Further, when a component is mentioned to be "coupled to", "supported by", and "in contact with" another component, it should be understood that it may be directly coupled to, supported by, connected to, supplied to, transferred to, and in contact with another component, but there may be other components therebetween.

The terms used in the present specification are used merely to describe the specific embodiments and are not intended to limit the present disclosure. Singular expressions include the plural unless the context clearly indicates otherwise.

In addition, it should be noted in advance that in the present specification, expressions such as upper, lower, front-rear direction, front, rear, etc. are described based on the illustrations in the drawings, and may be expressed differently if the orientation of the subject is changed. For the same reason, in the accompanying drawings, some components are exaggerated, omitted, or schematically shown, and the size of each component does not entirely reflect the actual size.

Furthermore, the terms containing ordinal numbers, such as first, second, etc., may be used to describe various components, but the components are not limited by such terms. These terms are used only to distinguish one component from another.

The meaning of "include" used in the present specification is intended to specify certain features, areas, integers, steps, operations, elements, and/or components, and is not intended to exclude the existence or the addition of other specific features, areas, integers, steps, operations, elements, components, and/or groups.

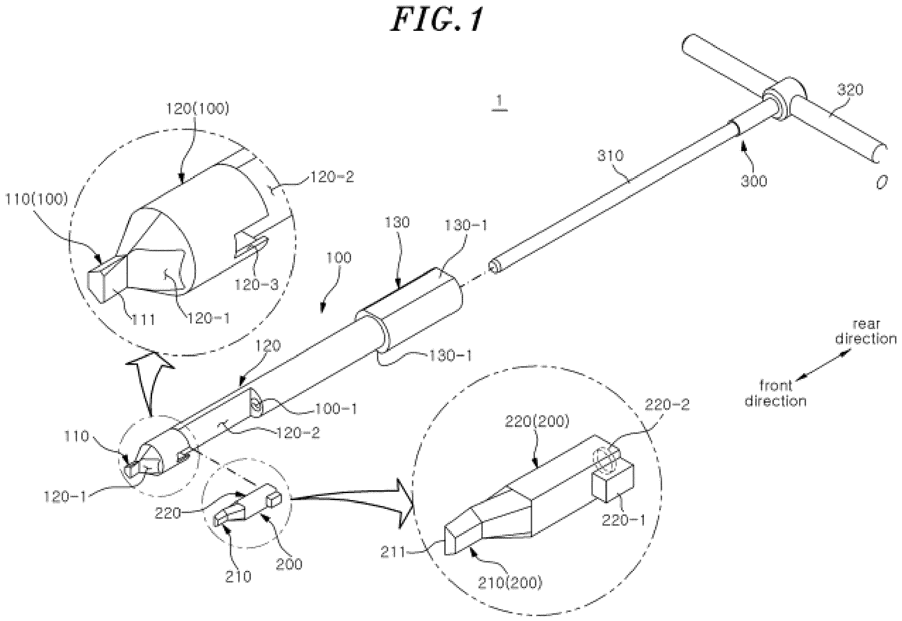

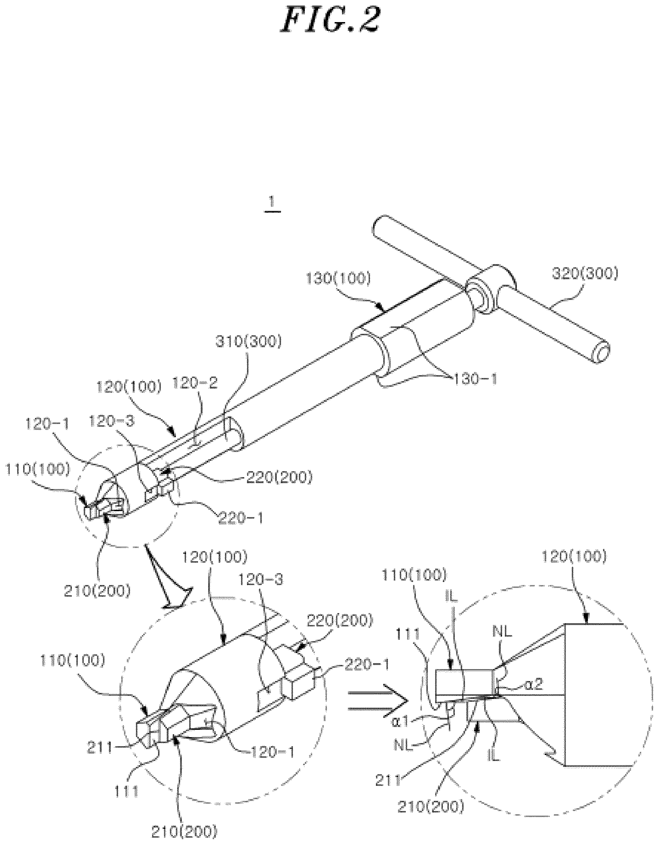

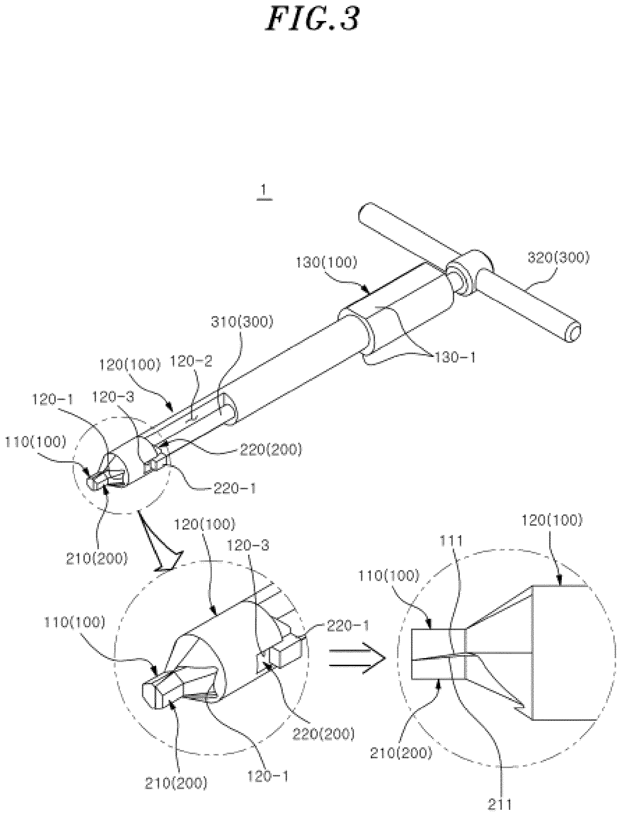

Hereinafter, the specific configuration of a screw removal device according to a first embodiment of the present disclosure will be described with reference to FIGS. 1 to 3. The screw removal device 1 is a device that removes a screw 2 from a screw coupling member 3 to which the screw 2 is coupled. The screw 2 to be removed by the screw removal device 1 may include a screw body 2-1 having threads 2-11 formed on at least a portion of its outer periphery and a screw head 2-2 connected to an upper part of the screw body 2-1 and having an insertion groove 2-21 formed therein. The screw removal device 1 may be configured such that at least a portion thereof is engaged with or disengaged from the insertion groove 2-21 of the screw 2. In other words, the screw removal device 1 may be configured such that at least a portion of the screw removal device 1 is press-fitted into the insertion groove 2-21 of the screw 2 or the press-fitting is released. In a state in which at least a portion of the screw removal device 1 is engaged with the insertion groove 2-21 of the screw 2, the screw 2 is rotated together with the screw removal device 1, so that the screw 2 is released from the screw coupling member 3 to be removed from the screw coupling member 3. The screw removal device 1 may include a body member 100, a wedge member 200, and a pressing member 300.

Referring to FIGS. 1 to 3, the body member 100 may support movement of the wedge member 200 and the pressing member 300. In other words, the wedge member 200 and the pressing member 300 may be movably disposed on the body member 100. In addition, a portion of the body member 100 may be inserted into the insertion groove 2-21 of the screw 2, and the portion of the body member 100 inserted into the insertion groove 2-21 of the screw 2 may engage a portion of the wedge member 200 through movement of the wedge member 200. The body member 100 may extend in the front-rear direction. The front-rear direction in which the body member 100 extends may be the direction in which the wedge member 200 and the pressing member 300 move. Further, the body member 100 may support the movement of the wedge member 200 and the pressing member 300 in the front-rear direction. In other words, the wedge member 200 and the pressing member 300 may be disposed to be movable in the front-rear direction on the body member 100.

A pressing member moving hole 100-1 may be formed in the body member 100. At least a portion of the pressing member 300 may be movably disposed in the pressing member moving hole 100-1. The pressing member moving hole 100-1 extends in the front-rear direction, and the pressing movement portion 310, which will be described later, may be disposed in the pressing member moving hole 100-1 to be movable in the front-rear direction. The pressing member moving hole 100-1 may be connected to an exposed space 120-2, which will be described later. A female thread (not shown) may be formed in the pressing member moving hole 100-1. In addition, the pressing movement portion 310 may be moved while rotating in the pressing member moving hole 100-1. The body member 100 may include a first groove insertion portion 110, a member middle portion 120, and a grip portion 130.

The first groove insertion portion 110 may be inserted into the insertion groove 2-21 of the screw 2. The first groove insertion portion 110 may have a polygonal shape when viewed in the front-rear direction. For example, when viewed in the front-rear direction, the first groove insertion portion 110 may have a trapezoidal shape. In other words, the first groove insertion portion 110 may have a polygonal pillar shape, such as a trapezoidal pillar. In addition, when the first groove insertion portion 110 and the second groove insertion portion 210 are viewed in the front-rear direction with the wedge member 200 placed in the engaged position, the first groove insertion portion 110 and the second groove insertion portion 210 may form a polygonal shape. For example, when the first groove insertion portion 110 and the second groove insertion portion 210 are viewed in the front-rear direction with the wedge member 200 placed in the engaged position, the first groove insertion portion 110 and the second groove insertion portion 210 may have a hexagonal shape or a star-like shape. In other words, when the wedge member 200 is placed in the engaged position, the first groove insertion portion 110 and the second groove insertion portion 210 may form a polygonal pillar such as a hexagonal pillar or a star-shaped pillar.

A first pressing guide surface 111 may be formed in the first groove insertion portion 110. The first pressing guide surface 111 may be an inclined surface. The first pressing guide surface 111 may be inclined such that the cross-section of the first groove insertion portion 110 perpendicular to the front-rear direction increases toward the front. In addition, the first pressing guide surface 111 may form a first angle α1, which is an acute angle between a normal line NL to the first pressing guide surface 111 and an imaginary line IL in the front-rear direction. Further, the first pressing guide surface 111 may be inclined such that the first angle α1 is larger than a second angle a2, which is an acute angle between a normal line NL to the second pressing guide surface 211 and the imaginary line IL in the front-rear direction and will be described later.

The member middle portion 120 may support movement of the wedge member 200. In addition, the member middle portion 120 may support movement of the pressing member 300 together with the grip portion 130. The member middle portion 120 may be connected to the first groove insertion portion 110. The member middle portion 120 may extend in the front-rear direction to support the movement of the wedge member 200 and the pressing member 300 in the front-rear direction. The wedge member moving hole 120-1, the exposed space 120-2, a moving guide groove 120-3, and a portion of the pressing member moving hole 100-1 may be formed in the member middle portion 120.

The wedge member moving hole 120-1 may be formed in the member middle portion 120 to be connected to the first pressing guide surface 111. The wedge member 200 may be movably disposed in the wedge member moving hole 120-1. The wedge member moving hole 120-1 may extend in the front-rear direction. In addition, the wedge member 200 may be disposed in the wedge member moving hole 120-1 to be movable in the front-rear direction.

The exposed space 120-2 may be formed in the member middle portion 120 to be connected to the wedge member moving hole 120-1 and to be exposed to the outside. The wedge member 200 and the pressing member 300 may be movably disposed in the exposed space 120-2. The exposed space 120-2 may extend in the front-rear direction. In addition, the wedge member 200 and the pressing member 300 may be disposed in the exposed space 120-2 to be movable in the front-rear direction.

The moving guide groove 120-3 may guide movement of the wedge member 200. The moving guide groove 120-3 may guide movement of a wedge member movement portion 220 included in the wedge member 200, which will be described later. The moving guide groove 120-3 may be connected to the wedge member moving hole 120-1 and the exposed space 120-2. The moving guide groove 120-3 may extend in the front-rear direction. In addition, a moving guide protrusion 220-1, which will be described later, formed on the wedge member movement portion 220, may be inserted into the moving guide groove 120-3 to be movable in the front-rear direction.

The grip portion 130 may support movement of the pressing member 300 and may be gripped by a user (not shown). The grip portion 130 may be connected to the member middle portion 120. The grip portion 130 may extend in the front-rear direction. Another portion of the pressing member moving hole 100-1 may be formed in the grip portion 130. The pressing movement portion 310 of the pressing member 300 may be disposed in the pressing member moving hole 100-1 of the grip portion 130 to be movable in the front-rear direction. A gripping surface 130-1 that a user can grip may be formed on the grip portion 130. The gripping surface 130-1 may be flat and may be provided in plural numbers. For example, two gripping surfaces 130-1 may be provided, and one of the two gripping surfaces 130-1 is formed on an upper side of the grip portion 130 and the other is formed on a lower side of the grip portion 130.

The wedge member 200 may be disposed to be movable in the front-rear direction with respect to the body member 100. The wedge member 200 may be placed in a disengaged position or an engaged position by moving in the front-rear direction. The disengaged position is a position where at least one of the first groove insertion portion 110 and the second groove insertion portion 210 is not pushed against each other in a direction perpendicular to at least one of the first pressing guide surface 111 and the second pressing guide surface 211. Further, the engaged position is a position where the first pressing guide surface 111 and the second pressing guide surface 211 are disposed to face each other, and at least one of the first groove insertion portion 110 and the second groove insertion portion 210 is pushed against each other in the direction perpendicular to at least one of the first pressing guide surface 111 and the second pressing guide surface 211. When the wedge member 200 is placed in the disengaged position with a portion of the body member 100 inserted into the insertion groove 2-21 of the screw 2, a portion of the wedge member 200 may not engage with a portion of the body member 100. In addition, when the wedge member 200 is placed in the engaged position with a portion of the body member 100 inserted into the insertion groove 2-21 of the screw 2, a portion of the wedge member 200 may engage with a portion of the body member 100. Further, a portion of the wedge member 200 and a portion of the body member 100 may be in close contact with the insertion groove 2-21 of the screw 2. In other words, a portion of the wedge member 200 and a portion of the body member 100 may be press-fitted into the insertion groove 2-21 of the screw 2. The wedge member 200 may extend in the front-rear direction. The wedge member 200 may include a second groove insertion portion 210 and a wedge member movement portion 220.

When the wedge member 200 is placed in the disengaged position, the second groove insertion portion 210 may not engage with the first groove insertion portion 110 such that the first groove insertion portion 110 of the body member 100 can be inserted into the insertion groove 2-21 of the screw 2. In addition, in a state in which the first groove insertion portion 110 is inserted into the insertion groove 2-21 of the screw 2, the second groove insertion portion 210 may engage with the first groove insertion portion 110 by placing the wedge member 200 in the engaged position. Further, at least a portion of the second groove insertion portion 210 and at least a portion of the first groove insertion portion 110 may be in close contact with the insertion groove 2-21 of the screw 2. In other words, when the wedge member 200 is placed in the engaged position, the second groove insertion portion 210 can be press-fitted into the insertion groove 2-21 of the screw 2 together with the first groove insertion portion 110.

The second groove insertion portion 210 may have a polygonal shape when viewed in the front-rear direction. For example, when viewed in the front-rear direction, the second groove insertion portion 210 may have a trapezoidal shape. In other words, the second groove insertion portion 210 may have a polygonal pillar shape, such as a trapezoidal pillar. In addition, when the second groove insertion portion 210 and the first groove insertion portion 110 are viewed in the front-rear direction with the wedge member 200 placed in the engaged position, the second groove insertion portion 210 and the first groove insertion portion 110 may have a polygonal shape. For example, when the second groove insertion portion 210 and the first groove insertion portion 110 are viewed in the front-rear direction with the wedge member 200 placed in the engaged position, the second groove insertion portion 210 and the first groove insertion portion 110 may have a hexagonal shape or a star-like shape. In other words, when the wedge member 200 is placed in the engaged position, the second groove insertion portion 210 and the first groove insertion portion 110 may form a polygonal pillar such as a hexagonal pillar or a star-shaped pillar.

A second pressing guide surface 211 may be formed in the second groove insertion portion 210. The second pressing guide surface 211 may be formed in the second groove insertion portion 210 to face the first pressing guide surface 111 of the first groove insertion portion 110. If the area where the second pressing guide surface 211 and the first pressing guide surface 111 face each other is called a facing area, the facing area at the engaged position of the wedge member 200 may be larger than the opposing area at the disengaged position of the wedge member 200. The second pressing guide surface 211 may be an inclined surface. The second pressing guide surface 211 may be inclined such that the cross-section of the second groove insertion portion 210 perpendicular to the front-rear direction becomes smaller toward the front. In addition, the second pressing guide surface 211 may form a second angle α2, which is an acute angle between a normal line NL to the second pressing guide surface 211 and the imaginary line II, in the front-rear direction. Further, the second pressing guide surface 211 may be inclined such that the second angle α2 is smaller than the first angle α1, which is an acute angle between the normal line to the first pressing guide surface 111 and an imaginary line II, in the front-rear direction.

The wedge member movement portion 220 may move together with the second groove insertion portion 210 in the front-rear direction with respect to the body member 100. The wedge member movement portion 220 may be connected to the second groove insertion portion 210. The wedge member movement portion 220 may extend in the front-rear direction. The wedge member movement portion 220 may be movably disposed in the wedge member moving hole 120-1 and the exposed space 120-2 of the body member 100. A moving guide protrusion 220-1 and a pressing movement portion contact groove 220-2 may be formed in the wedge member movement portion 220.

The moving guide protrusion 220-1 may be movably inserted into the moving guide groove 120-3 of the member middle portion 120 of the body member 100. The moving guide protrusion 220-1 may radially protrude further than a peripheral surface of the wedge member movement portion 220. The moving guide protrusion 220-1 not only guides the movement of the wedge member 200, but can also be used to move the wedge member 200 from the engaged position to the disengaged position.

One end of the pressing member 300 may be in contact with the pressing movement portion contact groove 220-2. For example, a front end of the pressing movement portion 310 included in the pressing member 300 may contact the pressing movement portion contact groove 220-2.

The pressing member 300 may be disposed on the body member 100 to move the wedge member 200 in the front-rear direction. A portion of the pressing member 300 may be disposed on the body member 100 to move in the front-rear direction while rotating. The pressing member 300 may include a pressing movement portion 310 and a handle portion 320.

The pressing movement portion 310 may be disposed to be movable in the front-rear direction on the body member 100 to press the wedge member 200. The pressing movement portion 310 may be inserted through the pressing member moving hole 100-1 of the body member 100 and may be disposed to be movable in the front-rear direction. A male thread that engages with the female thread formed in the pressing member moving hole 100-1 may be formed on the pressing movement portion 310. The pressing movement portion 310 may be moved in the front-rear direction while rotating in the pressing member moving hole 100-1 by a rotational force applied by the user through the handle portion 320. In other words, the pressing movement portion 310 may be moved forward when rotated in one direction by a rotational force applied by the user through the handle portion 320, and may be moved rearward when rotated in the other direction opposite to the one direction. In addition, the front end of the pressing movement portion 310 may be in contact with the pressing movement portion contact groove 220-2 of the wedge member movement portion 220 of the wedge member 200. Further, the wedge member 200 may be moved by movement of the pressing movement portion 310. For example, when the pressing movement portion 310 moves forward, the wedge member 200 may be moved forward so that the wedge member 200 is moved from the disengaged position to the engaged position.

The handle portion 320 may be configured to apply a rotational force to the pressing movement portion 310. In other words, when the user grips the handle portion 320 and rotates the handle portion 320, the pressing movement portion 310 may rotate together with the handle portion 320. The handle portion 320 may extend from the pressing movement portion 310 to form a predetermined angle with the front-rear direction. For example, the handle portion 320 may extend from a rear end portion of the pressing movement portion 310 to form a right angle with the front-rear direction.

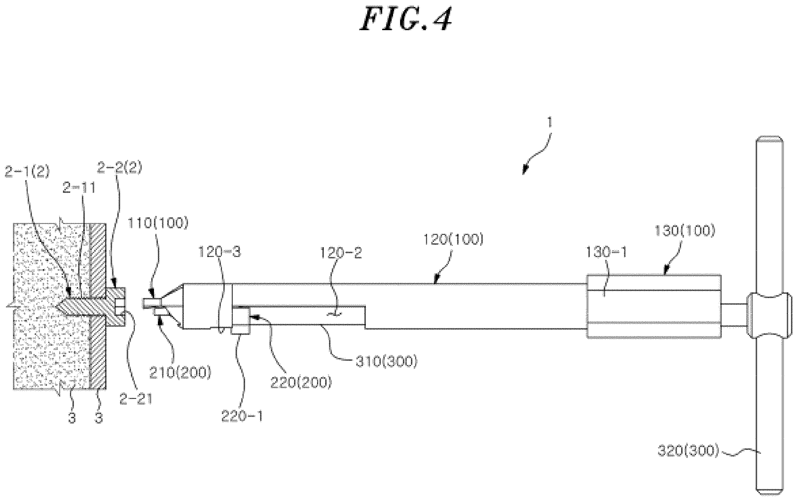

Hereinafter, with reference to FIGS. 4 to 7, the operation and effect of the screw removal device 1 according to the first embodiment of the present disclosure having the above-described configuration will be described.

Referring to FIG. 4, the user can move the screw removal device 1 to a location where the screw 2 to be removed is coupled to the screw coupling member 3. In addition, the wedge member 200 of the screw removal device 1 is placed in the disengaged position, and the front end of the pressing movement portion 310 of the pressing member 300 may be in contact with the pressing movement portion contact groove 220-2 of the wedge member movement portion 220 of the wedge member 200.

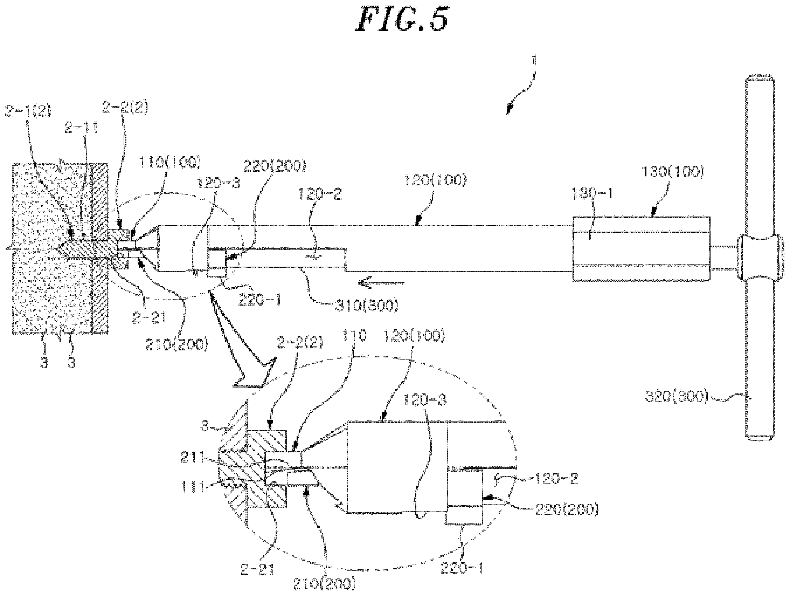

Referring to FIG. 5, after moving the screw removal device 1 to the location where the screw 2 to be removed is coupled to the screw coupling member 3, the user can grip the grip portion 130 of the body member 100 of the screw removal device 1 and the handle portion 320 of the pressing member 300. In this state, the user can insert the groove insertion portion 110 of the body member 100 into the insertion groove 2-21 of the screw 2.

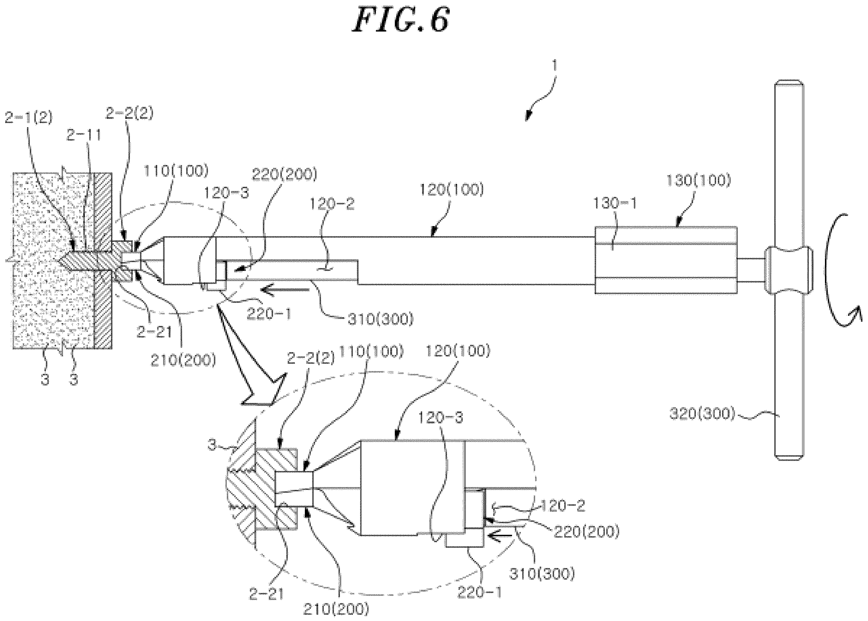

Referring to FIG. 6, in the state in which the groove insertion portion 110 of the body member 100 is inserted into the insertion groove 2-21 of the screw 2, the user can rotate the pressing member 300 using the handle portion 320 to move the pressing member 300 forward. As the pressing member 300 moves forward, the wedge member 200 moves forward so that the wedge member 200 can be moved from the disengaged position to the engaged position. When the wedge member 200 is placed in the engaged position, the first pressing guide surface 111 of the first groove insertion portion 110 of the body member 100 and the second pressing guide surfaces 211 of the second groove insertion portion 210 of the wedge member 200 can be arranged to face each other. In addition, at least one of the first groove insertion portion 110 and the second groove insertion portion 210 can be pushed against each other in the direction perpendicular to at least one of the first pressing guide surface 111 and the second pressing guide surface 211. Further, at least a portion of the first groove insertion portion 110 and at least a portion of the second groove insertion portion 210 can be in close contact with the insertion groove 2-21 of the screw 2. In other words, the first groove insertion portion 110 and the second groove insertion portion 210 can be press-fitted into the insertion groove 2-21 of the screw 2.

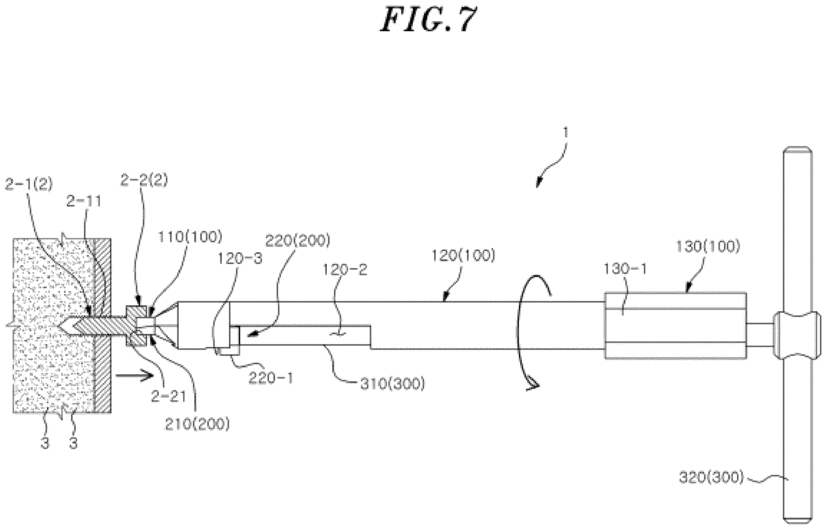

Referring to FIG. 7, in the state in which at least a portion of the first groove insertion portion 110 of the body member 100 and at least a portion of the second groove insertion portion 210 of the wedge member 200 are in close contact with the insertion groove 2-21 of the screw 2, the user can rotate the screw removal device 1 in the direction that loosens the screw 2. By rotating the screw removal device 1 in the direction that loosens the screw 2, the screw 2 can be released from the screw coupling member 3 to be removed from the screw coupling member 3.

After removing the screw 2 from the screw coupling member 3, the user can rotate the pressing member 300 using the handle portion 320 to move the pressing member 300 rearward. As the pressing member 300 moves rearward, the front end of the pressing movement portion 310 of the pressing member 300 does not contact the pressing movement portion contact groove 220-2 of the wedge member movement portion 220 of the wedge member 200 and can be moved away from the wedge member movement portion 220.

In this state, the user can apply a rearward force to the moving guide protrusion 220-1 of the wedge member 200 to move the wedge member 200 to the disengaged position. In addition, the screw 2 and the screw removal device 1 can be separated. In other words, after separating the second groove insertion portion 210 of the wedge member 200 from the insertion groove 2-21 of the screw 2, by separating the first groove insertion portion 110 of the body member 100 from the insertion groove 2-21 of the screw 2, the screw 2 and the screw removal device 1 can be separated.

In this way, the screw removal device 1 according to the first embodiment of the present disclosure can increase the contact force with the insertion groove 2-21 of the screw 2 when removing the screw 2. In other words, the first groove insertion portion 110 and the second groove insertion portion 210 of the screw removal device 1 can be press-fitted into the insertion groove 2-21 of the screw 2. Accordingly, the screw 2 can be removed from the screw coupling member 3 without damaging the insertion groove 2-21, and the screw 2 can be easily removed from the screw coupling member 3 even if the insertion groove 2-21 has already been damaged.

Meanwhile, in addition to such configurations, according to a second embodiment of the present disclosure, the size of the second groove insertion portion 210 may be smaller than the size of the first groove insertion portion 110, a first guide surface 112 may be further formed in the first groove insertion portion 110, and a second guide surface 212 may be further formed in the second groove insertion portion 210.

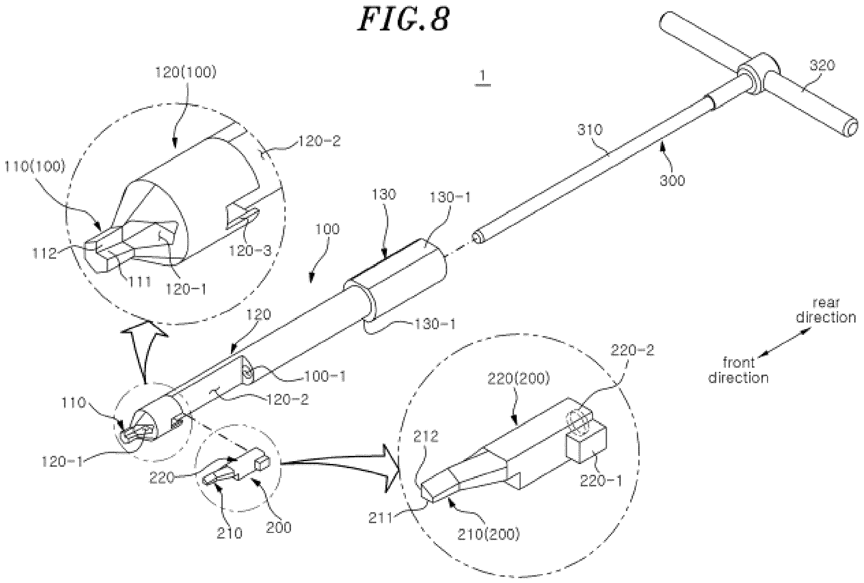

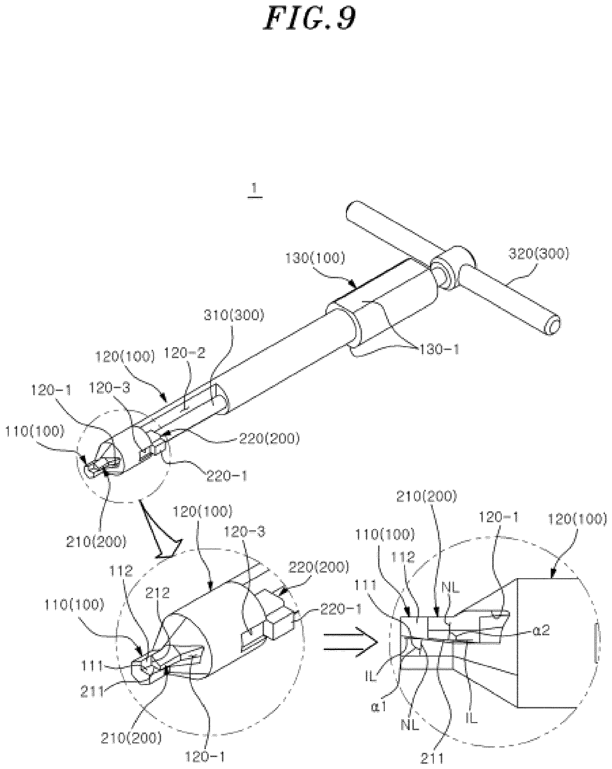

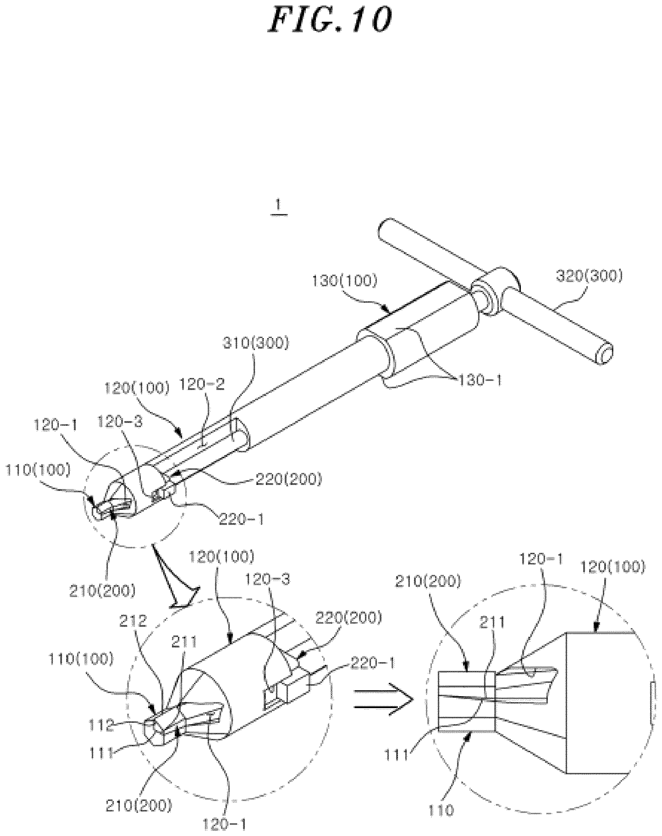

Hereinafter, with reference to FIGS. 8 to 12, a second embodiment of the present disclosure will be described. In describing the second embodiment of the present disclosure, when compared to the above-described first embodiment, there are differences in that the size of the second groove insertion portion 210 may be smaller than the size of the first groove insertion portion 110, a first guide surface 112 is further formed in the first groove insertion portion 110, and a second guide surface 212 is further formed in the second groove insertion portion 210, so these differences will be mainly described, and the same description and reference numerals will be referred to the above-described embodiment.

Referring to FIGS. 8 to 10, the size of the second groove insertion portion 210 may be smaller than the size of the first groove insertion portion 110. For example, the second groove insertion portion 210 may be 1/4 the size of the hexagonal pillar or star-shaped pillar, and the first groove insertion portion 110 may be 3/4 the size of the hexagonal pillar or star-shaped pillar. In other words, the size of the second groove insertion portion 210 may be 1/3 the size of the first groove insertion portion 110.

The first guide surface 112 may be connected to the first pressing guide surface 111 to form a predetermined angle with the first pressing guide surface 111. For example, the first guide surface 112 may be connected to the first pressing guide surface 111 to form a right angle to the first pressing guide surface 111 when viewed in the front-rear direction. The first guide surface 112 may guide the movement of the wedge member 200 while facing the second guide surface 212. The wedge member moving hole 120-1 may be connected to the first pressing guide surface 111 and the first guide surface 112.

The second guide surface 212 may be connected to the second pressing guide surface 211 to form a predetermined angle with the second pressing guide surface 211 and may face the first guide surface 112. For example, the second guide surface 212 may be connected to the second pressing guide surface 211 to form a right angle with the second pressing guide surface 211. The second guide surface 212 may guide the movement of the wedge member 200 together with the first guide surface 112.

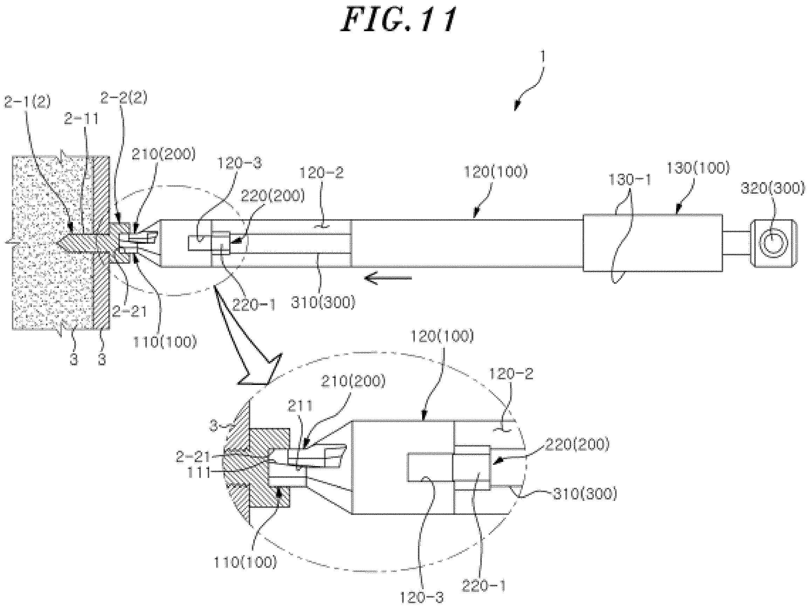

Referring to FIG. 11, in the state in which the wedge member 200 of the screw removal device 1 is placed in the disengaged position, the user can insert the first groove insertion portion 110 of the body member 100 into the insertion groove 2-21 of the screw 2.

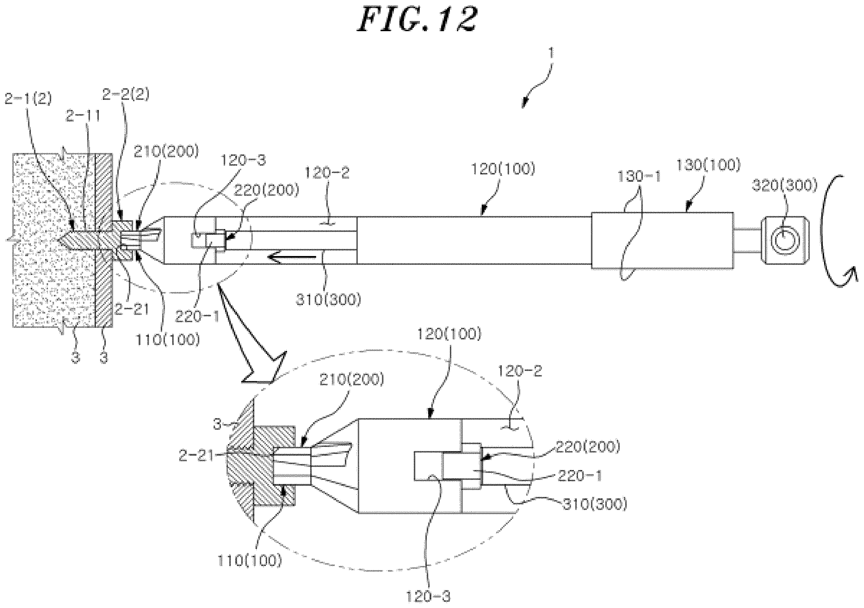

Referring to FIG. 12, in the state in which the groove insertion portion 110 of the body member 100 is inserted into the insertion groove 2-21 of the screw 2, the user can rotate the pressing member 300 using the handle portion 320 to move the pressing member 300 forward. As the pressing member 300 moves forward, the wedge member 200 moves forward so that the wedge member 200 can be moved from the disengaged position to the engaged position. When the wedge member 200 is placed in the engaged position, the first pressing guide surface 111 of the first groove insertion portion 110 of the body member 100 and the second pressing guide surfaces 211 of the second groove insertion portion 210 of the wedge member 200 can be arranged to face each other. In addition, at least one of the first groove insertion portion 110 and the second groove insertion portion 210 can be pushed against each other in the direction perpendicular to at least one of the first pressing guide surface 111 and the second pressing guide surface 211. Further, at least a portion of the first groove insertion portion 110 and at least a portion of the second groove insertion portion 210 can be in close contact with the insertion groove 2-21 of the screw 2. In other words, the first groove insertion portion 110 and the second groove insertion portion 210 may be press-fitted into the insertion groove 2-21 of the screw 2.

In a state in which at least a portion of the first groove insertion portion 110 of the body member 100 and at least a portion of the second groove insertion portion 210 of the wedge member 200 are in close contact with the insertion groove 2-21 of the screw 2, the user can rotate the screw removal device 1 in the direction that loosens the screw 2. By rotating the screw removal device 1 in the direction that loosens the screw 2, the screw 2 can be released from the screw coupling member to be removed from the screw coupling member 3.

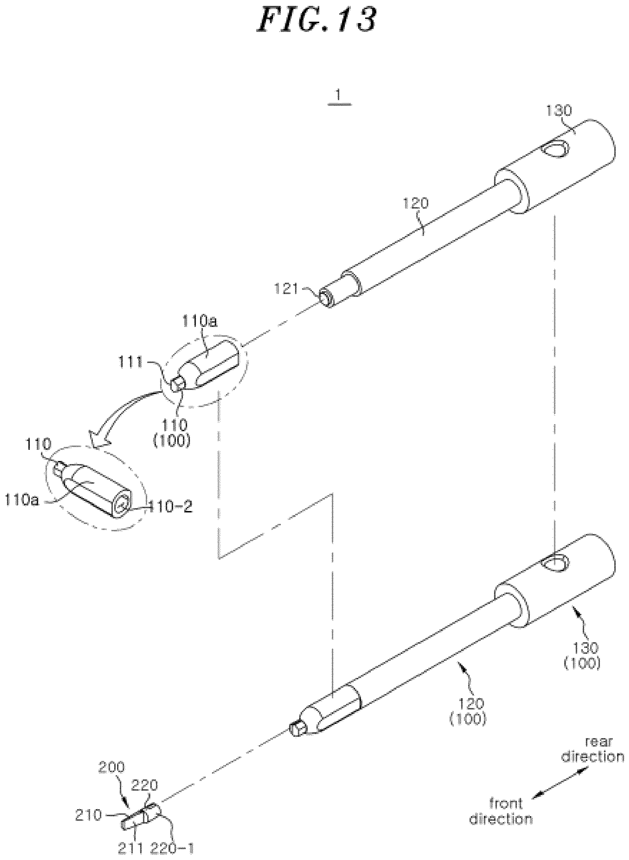

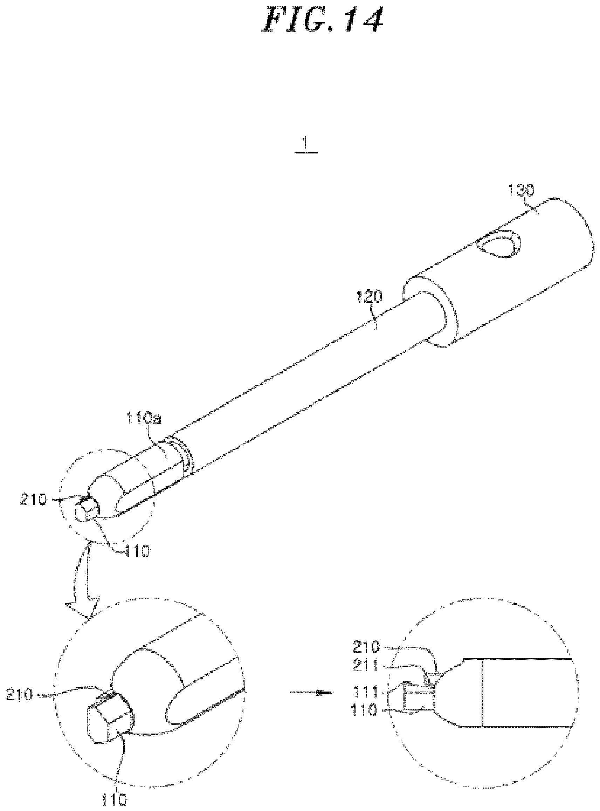

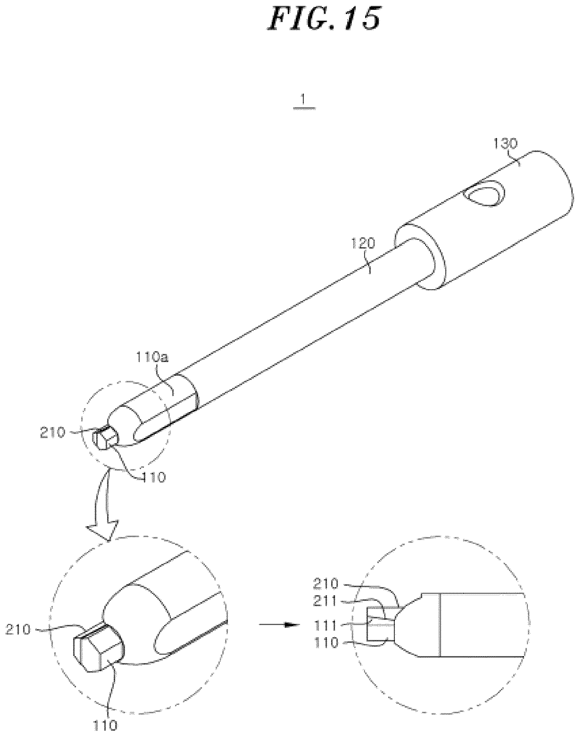

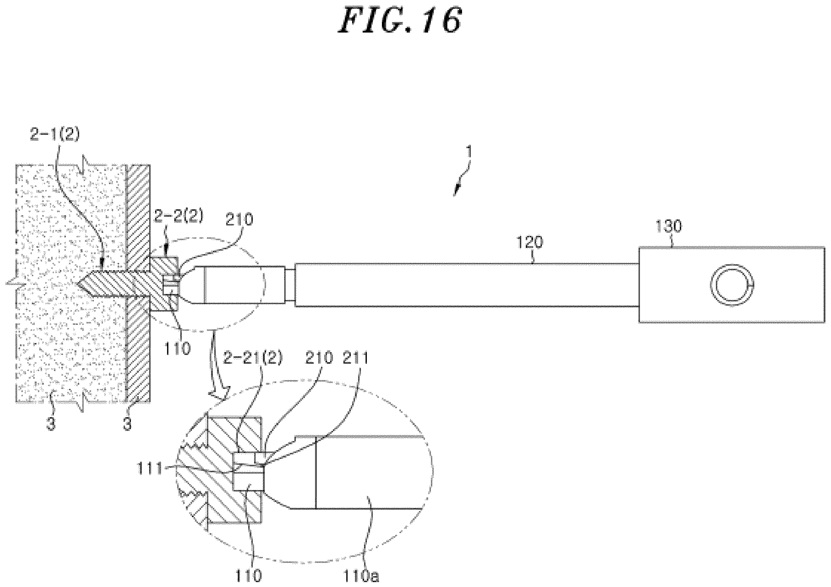

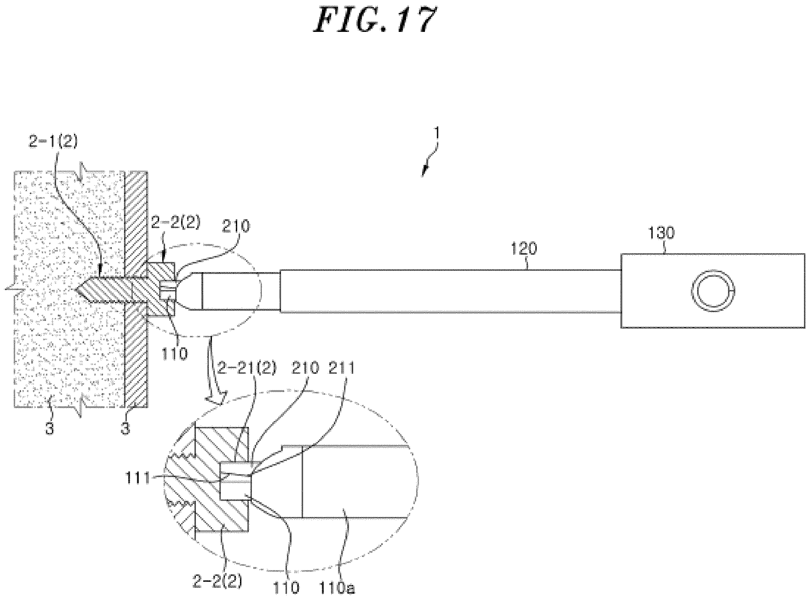

Hereinafter, with reference to FIGS. 13 to 17, a third embodiment of the present disclosure will be described. In describing the third embodiment of the present disclosure, when compared to the above-described first embodiment, there are differences in that the pressing member 300 can be removed, and a first groove insertion body 110a in which the first groove insertion portion 110 is formed is selectively connected to the member middle portion 120. Further, there are differences in that the second groove insertion portion 210 is provided in various forms and is used based on the shape and size of the insertion groove 2-21 of the screw 2, so these differences will be mainly described, and the same description and reference numerals will be referred to the above-described embodiment. Further, the pressing connection part 121 may be disposed in a pressing connection part moving hole 100-2 formed in the first groove insertion body 110a to move in the front-reat direction.

Referring to FIGS. 13 to 17, in the screw removal device 1 according to the third embodiment of the present invention, the first groove insertion portion 110 may be formed in the first groove insertion body 110a, and the first groove insertion body 110a may be selectively connected to the member middle portion 120. The pressing connection part 121 to which the first groove insertion body 110a is selectively connected may be formed at the end of the member middle portion 120. In other words, the member middle portion 120 and the first groove insertion body 110a may be seperated from or connected to each other. The pressing connection part moving hole 100-2 may be formed in the first groove insertion body 110a so that the pressure connection part 121 may be inserted. The pressing connection part 121 may press the wedge member 200 by moving with respect to the first groove insertion body 110a in the front-rear direction, and the pressing connection part 121 is disposed on the pressing connection part moving hole 100-2. In other words, the screw removal device 1 may not include the pressing member 300. Further, the pressing connection part 121 may be formed with a male thread that meshes with the femal thread formed in the pressing connection part moving hole 100-2.

In addition, a plurality of the second groove insertion portion 210 of the wedge member 200 may be provided based on the shape and size of the insertion groove 2-21 of the screw 2. For example, when the member middle portion 120 and the first groove insertion body 110a are seperated, any one of the plurality of wedge members 200 connected to the first groove insertion portion 110 that matches the shape and size of the insertion groove 2-21 of the screw 2 can be selected.

Although the embodiments of the present disclosure have been described above as specific embodiments, these are merely examples. The present disclosure is not limited to the above and should be construed as having the broadest scope according to the technical idea disclosed in the present specification. Those skilled in the art may implement a pattern of a shape not specified above by combining/substituting the disclosed embodiments, without departing from the scope of the present disclosure. In addition, those skilled in the art may easily change or modify the embodiments disclosed based on the present specification, and it is clear that such changes or modifications also fall within the scope of the present disclosure.

## Claims

1. A screw removal device comprising:
a body member including a first groove insertion portion and extending in a front-rear direction;
a wedge member including a second groove insertion portion and disposed to be movable in the front-rear direction with respect to the body member; and
a pressing member disposed on the body member to move the wedge member in the front-rear direction,
wherein a first pressing guide surface is formed in the first groove insertion portion, and a second pressing guide surface is formed in the second groove insertion portion to face the first pressing guide surface, and
the wedge member is placed in a disengaged position in which at least one of the first groove insertion portion and the second groove insertion portion are not pushed against each other in a direction perpendicular to at least one of the first pressing guide surface and the second pressing guide surface, or in an engaged position in which the first pressing guide surface and the second pressing guide surface are arranged to face each other, and at least one of the first groove insertion portion and the second groove insertion portion are pushed against each other in the direction perpendicular to at least one of the first pressing guide surface and the second pressing guide surface.

2. The screw removal device of claim 1, wherein when the first groove insertion portion and the second groove insertion portion are viewed in the front-rear direction with the wedge member placed in the engaged position, the first groove insertion portion and the second groove insertion portion form a polygonal shape.

3. The screw removal device of claim 2, wherein the polygonal shape is hexagonal or star-shaped.

4. The screw removal device of any one of claims 1 to 3, wherein the first pressing guide surface and the second pressing guide surface are inclined such that a first angle, which is an acute angle between a normal to the first pressing guide surface and an imaginary line in the front-rear direction, is greater than a second angle which is an acute angle between a normal to the second pressing guide surface and the imaginary line in the front-rear direction.

5. The screw removal device of any one of claims 1 to 4, wherein in the body member, a wedge member moving hole which is connected to the first pressing guide surface and in which the wedge member is movably disposed is formed.

6. The screw removal device of claim 5, wherein in the body member, an exposed space which is connected to the wedge member moving hole and exposed to an outside and in which the wedge member and the pressing member are movably disposed is formed.

7. The screw removal device of claim 6, wherein in the body member, a pressing member moving hole which is connected to the exposed space and in which at least a portion of the pressing member is movably disposed is formed.

8. The screw removal device of claim 7, wherein the body member further includes:
a member middle portion which is connected to the first groove insertion portion and in which the wedge member moving hole, the exposed space, and a portion of the pressing member moving hole are formed; and
a grip portion which is connected to the member middle portion and in which another portion of the pressing member moving hole is formed.

9. The screw removal device of claim 7 or 8, wherein the wedge member further includes a wedge member movement portion connected to the second groove insertion portion and movably disposed in the wedge member moving hole and the exposed space.

10. The screw removal device of claim 9, wherein a moving guide groove, which is connected to the wedge member moving hole and the exposed space and guides the movement of the wedge member movement portion, is further formed in the body member, and
a moving guide protrusion movably inserted into the moving guide groove is formed on the wedge member movement portion.

11. The screw removal device of claim 10, wherein the moving guide protrusion protrudes radially further than a peripheral surface of the wedge member movement portion.

12. The screw removal device of any one of claims 1 to 11, wherein the pressing member includes:
a pressing movement portion disposed to be movable in the front-rear direction on the body member to press the wedge member; and
a handle portion extending from the pressing movement portion to form a predetermined angle with the front-rear direction.

13. The screw removal device of any one of claims 1 to 12, wherein a size of the second groove insertion portion is smaller than a size of the first groove insertion portion.

14. The screw removal device of claim 13, wherein a first guide surface is further formed in the first groove insertion portion and connected to the first pressing guide surface to form a predetermined angle with the first pressing guide surface, and
a second guide surface is further formed in the second groove insertion portion and connected to the second pressing guide surface to form a predetermined angle with the second pressing guide surface, the second guide surface facing the first guide surface.

15. A screw removal device comprising:
a body member including a first groove insertion portion and extending in a front-rear direction; and
a wedge member including a second groove insertion portion and disposed to be movable in the front-rear direction with respect to the body member,
wherein a first pressing guide surface is formed in the first groove insertion portion, and a second pressing guide surface is formed in the second groove insertion portion to face the first pressing guide surface, and
the wedge member is placed in a disengaged position in which at least one of the first groove insertion portion and the second groove insertion portion are not pushed against each other in a direction perpendicular to at least one of the first pressing guide surface and the second pressing guide surface, or in an engaged position in which the first pressing guide surface and the second pressing guide surface are arranged to face each other, and at least one of the first groove insertion portion and the second groove insertion portion are pushed against each other in the direction perpendicular to at least one of the first pressing guide surface and the second pressing guide surface.

16. The screw removal device of claim 15, wherein the wedge member is arranged to be selectively connected to at least of the body member, and
the body member includes a pressing connection part that presses the wedge member to move in the front-rear direction by moving in the front-rear direction.
